# EUROPEAN PATENT APPLICATION

(11) **EP 4 837 517 A1**
(43) Date of publication of application: **11.11.2026**
(21) Application number: 25751654.2
(22) Date of filing: 07.02.2025
(51) Int. Cl.: A61B 17/11

(54) **VASCULAR ANASTOMAT ASSEMBLY**

(30) Priority: 09.02.2024 CN 202410179068; 29.10.2024 CN 202411523345
(71) Applicant: Teda International Cardiovascular Hospital, Binhai New Area, Tianjin 300457 (CN)
(72) Inventor: LIU, Xiaocheng, Tianjin 300457 (CN)
(74) Representative: Gulde & Partner
(86) International application number: PCT/CN2025/076220
(87) International publication number: WO 2025/168055

(57) **Abstract**

A vascular anastomat assembly (1), comprising an application apparatus (100) and a vascular interface assembly (200) releasably connected to the application apparatus (100). The application apparatus (100) comprises a piercing assembly (40) movably arranged within a rod portion (22) of a handle (10), the piercing assembly (40) comprising a piercing portion to pierce a vascular wall, and the piercing portion being movable between a retraction position and an extension position; and a cutting assembly (20) movably arranged within a sheath (30) of the handle (10), the cutting assembly (20) being movable between a retraction position and a cutting position for cutting an incision on the vascular wall. The vascular interface assembly (200) comprises a main body portion (212); a clamping member (230) arranged at a distal end portion of the main body portion (212), the clamping member (230) being capable of being triggered to be in an open state; a flange (221) arranged at a periphery of the main body portion (212); and a trigger member (220), the trigger member (220) being configured to trigger the clamping member (230), such that the clamping member (230) is in the open state to clamp the vascular wall between the clamping member (230) and the flange (221) in a sealing manner.

## Description

### TECHNICAL FIELD

The present disclosure relates to the technical field of vascular surgery, and in particular, to a vascular anastomosis assembly utilized in a synthetic vascular synchronous anastomosis surgery.

### BACKGROUND

As is well known, aortic bypass graft surgery is a conventional surgical technique for treating localized aortic lesions. The method involves using a synthetic vascular graft as a vascular substitute to connect the proximal end and the distal end of the blood vessel in the diseased segment, forming a vascular bypass that allows the blood flow to bypass the diseased segment and reach the distal end, thereby restoring blood supply at the distal end, alleviating ischemic symptoms at the distal end, and saving the patient's life. Currently, such surgery has to be performed by using a known surgical scalpel. A longitudinal incision is first made in the side wall of the aorta occluded by a side wall clamp, and then the end of the synthetic vascular graft is anastomosed to the incision in the aorta by using surgical needles and sutures. However, this conventional surgical technique is complicated, prone to bleeding, highly invasive, highly risky, and difficult to perform.

To solve the above problems, a combined mechanical device capable of creating an opening in the aorta and installing a synthetic vascular graft has emerged. The working process of this combined mechanical device is generally as follows: first, an opening is made in the aorta; then, part of a wall-clamping mechanism is inserted into the incision, and the wall-clamping mechanism is triggered at an appropriate time to clamp the inner and outer walls of the incision, thereby achieving anastomosis between the incision in the vascular wall and the synthetic vascular graft.

However, existing combined mechanical devices suffer from problems such as complex wall-clamping structure and inconvenient wall-clamping operation.

### SUMMARY

In accordance with an embodiment of the present disclosure, a vascular anastomosis assembly is provided. The vascular anastomosis assembly includes an applicator device and a vascular interface assembly. The vascular interface assembly is releasably connected to the applicator device, so as to be detached from the applicator device during the anastomosis process and sealingly clamped and held onto a vascular wall. With the above structure, the vascular anastomosis assembly enables rapid and safe vascular bridging, drainage and other procedures without the need of blood flow occlusion or complicated operations for vascular wall suturing. It's suitable for minimally invasive surgery and avoids excessive larger incisions.

According to an embodiment, the applicator device includes a handle; a hollow sheath extending from the handle; a piercing assembly movably disposed within the sheath of the handle, the piercing assembly including a piercing member configured to pierce a vascular wall, and the piercing member being movable between a retracted position and an extended position; and a cutting assembly movably disposed within the sheath of the handle, the cutting assembly being movable between a retracted position and a cutting position, wherein in the extended position, the cutting assembly is rotatable, by a predetermined angle, relative to the handle to cut out an incision in the vascular wall. Accordingly, the applicator device can be operated by a single surgeon to conveniently cut out an incision in the vascular wall and release the vascular interface assembly into the incision, thereby completing the operation of the vascular anastomosis assembly.

In an embodiment, the vascular interface assembly is releasably disposed at a distal end of the applicator device, and includes a main body portion, a synthetic vascular graft extending from a proximal end of the main body portion, a clamping member disposed at a distal end of the main body portion and triggerable to an expanded state, a flange disposed on an outer periphery of the main body portion, and a triggering member configured to trigger the clamping member to the expanded state such that the vascular wall is sealingly clamped between the clamping member and the flange. When the vascular interface assembly is mounted on the applicator device, in the extended position, the piercing member of the piercing assembly is extended distally beyond the vascular interface assembly, and the cutting assembly is extended distally beyond the vascular interface assembly. In this way, with the arrangement of the releasable vascular interface assembly, it allows the surgeons to select vascular interface assemblies of different sizes according to patient conditions, thereby facilitating surgical operations.

The vascular anastomosis assembly of the present disclosure realizes rapid vascular anastomosis and bypass graft establishment without vascular incision, suturing and blood flow occlusion, and is also suitable for minimally invasive surgical procedures. The instrument features rapid and convenient operations as well as reduced surgical risks, and achieves substantial improvements over conventional surgical operations.

### BRIEF DESCRIPTION OF DRAWINGS

For clearer illustration of the technical solutions of the embodiments of the present disclosure, the accompanying drawings for the embodiments of the present disclosure are briefly introduced below. The accompanying drawings merely illustrate certain embodiments of the present disclosure, and are not intended to limit all the embodiments of the present disclosure thereto.
Fig. 1 illustrates a perspective view of a vascular anastomosis assembly according to the present disclosure;
Fig. 2 is an exploded perspective view illustrating the vascular anastomosis assembly shown in Fig. 1;
Fig. 3 is a partial cross-sectional view of an applicator device with a vascular interface assembly removed;
Fig. 4 is an exploded perspective view of a handle;
Fig. 5 is a perspective view illustrating a sheath;
Fig. 6 is a perspective view illustrating a cutting assembly;
Fig. 7 is a perspective view illustrating an embodiment of a cutting head;
Fig. 8 is a perspective view illustrating another embodiment of the cutting head;
Fig. 9A is a perspective view illustrating a further embodiment of a cutting head;
Fig. 9B is a perspective view illustrating still another embodiment of a cutting head;
Fig. 10 is a perspective view illustrating a piercing assembly;
Figs. 11A to 11E are views illustrating different embodiments of a piercing tip of a piercing assembly;
Fig. 12 is an overall perspective view illustrating a vascular interface assembly;
Fig. 13 is an exploded perspective view illustrating a vascular interface assembly;
Fig. 14 is a perspective view illustrating a triggering member;
Fig. 14A is a perspective view illustrating another embodiment of a triggering member;
Fig. 14B is a perspective view illustrating a further embodiment of a triggering member;
Fig. 14C is a perspective view illustrating a threaded sleeve engaged with a triggering member;
Fig. 15 is a perspective view illustrating a vascular interface assembly with a clamping member in an expanded state, from which a synthetic vascular graft portion is omitted;
Fig. 15A is a perspective view of an assembly adopting the cutting head of Fig. 9B;
Fig. 16 is a perspective view illustrating another embodiment of a clamping member;
Fig. 17 is a perspective view illustrating a further embodiment of a clamping member;
Fig. 18 is a view illustrating another embodiment of a main body portion of a vascular interface assembly; and
Figs. 19 to 23 are views illustrating a vascular interface assembly of an obliquely inserted vascular anastomosis assembly and constituent parts thereof.

### DETAILED DESCRIPTION

The technical solutions of the embodiments of the present disclosure are clearly and completely described below in conjunction with the accompanying drawings of the embodiments, so that the objectives, technical solutions and advantages of the present disclosure can be more apparent. Identical reference numerals in the drawings denote identical components. It should be noted that the described embodiments constitute a part of the embodiments of the present disclosure, rather than all the embodiments. All other embodiments obtained by a person of ordinary skill in the art based on the described embodiments of the present disclosure without creative labor shall fall within the protection scope of the present disclosure.

Unless otherwise defined, all technical and scientific terms used herein shall have the ordinary meanings understood by a person of ordinary skill in the art to which the present disclosure pertains. The terms "first", "second" and similar words used in the specification and claims of the present disclosure do not indicate any order, quantity or importance, but are merely used to distinguish different constituent components. Similarly, terms such as "one" or "a/an" do not necessarily denote a quantitative limitation. Terms such as "include" or "comprise" mean that the elements or objects preceding such terms cover the elements or objects or equivalents listed after the terms, without excluding other elements or objects. Terms such as "connect" or "couple" are not limited to physical or mechanical connection, and may include electrical connection, whether direct or indirect.

Directional terms used in the following specification and claims are defined as follows: the terms "proximal", "proximal direction", "proximal end" and the like refer to the side or direction close to a medical operator such as a surgeon when the vascular anastomosis assembly is in use; the terms "distal", "distal direction", "distal end" and the like refer to the side or direction toward or close to a lesion or a patient to be treated. In addition, the term "longitudinal direction" as used in the description below and the claims generally refers to the length direction of a described component or feature, and a circumferential direction refers to the direction surrounding the longitudinal direction.

When local lesions occur in arterial or venous blood vessels, it is usually necessary to establish a bypass vessel, i.e., the so-called bypass grafting, to route around the diseased segment and restore blood supply. However, such bypass grafting involves relatively complex surgical procedures, such as thoracotomy, vascular incisions and drainage, resulting in large incisions that slow down postoperative recovery of patients. Such surgical procedure with relatively complicated operative approach imposes higher requirements on the surgical experience and skills of surgeons. Moreover, due to the prolonged operation time, it poses significant challenges to both surgeons and patients.

According to the present disclosure, a vascular anastomosis assembly capable of rapidly bridging a synthetic vascular graft to a large blood vessel, such as the aorta or veins, is provided. The vascular anastomosis assembly avoids forming severe trauma or large incisions on patients, thereby simplifying the surgical procedures, shortening the operation time, and reducing the operational difficulty for surgeons. In addition, adopting the vascular anastomosis assembly of the present disclosure allows for flexible selection of vascular bridging positions according to surgical requirements without distance limitations.

According to the present disclosure, it provides a vascular anastomosis assembly, including an applicator device and a vascular interface assembly. The vascular interface assembly is releasably engaged onto the applicator device, so as to be installed into an incision of a blood vessel by operating the applicator device. The vascular interface assembly can be sealingly fixed at the vascular incision, and the vascular interface assembly is provided with a synthetic vascular graft to realize vascular bridging.

The applicator device may include a cutting assembly. By operating the applicator device, the cutting assembly can form an incision in the blood vessel. Then, through further operation of the applicator device, the vascular interface assembly can be placed into the formed incision. Thus, the entire surgical procedure can be completed simply by operating the applicator device, and the whole process is simplified.

Advantageously, before forming the incision, a piercing assembly is utilized first to pierce the vascular wall to provide an anchor point for the subsequent cutting operation. The piercing assembly can be accommodated within the applicator device, and can be extended out of the applicator device when needed to pierce the blood vessel.

Preferably, the piercing assembly includes a sharp tip to facilitate piercing of the vascular wall. In addition, the piercing assembly further includes a barb structure capable of hooking onto the vascular wall, so as to provide an anchor point for the cutting operation and allow the excised portion of the vascular wall to be removed after the cutting operation is completed subsequently.

For example, the vascular interface assembly includes a hollow, cylindrical main body portion, so as to be engaged into the incision in the vascular wall formed by the cutting assembly. In order to achieve sealing and fixation, the vascular interface assembly clamps the vascular wall around the incision. Thus, the vascular interface assembly may include a clamping member disposed at a distal portion of the cylindrical main body portion and a flange disposed at a proximal portion of the cylindrical main body portion. The clamping member and the flange cooperate with each other to clamp and hold the vascular wall. A sealing and hemostatic structure, such as a hemostatic pad, may be disposed on a distal end surface of the flange, to further enhance the sealing performance.

To facilitate insertion of the vascular interface assembly into the incision in the vascular wall, during application, the clamping member is in a retracted position, i.e., the clamping member is retracted within the cylindrical main body portion of the vascular interface assembly. After the vascular interface assembly is inserted into the incision, for example, when the flange is abutted against the outer side of the vascular wall, the clamping member is triggered to switch to an expanded position through an operation of the applicator device, thereby achieving clamping of the vascular wall. The expansion of the clamping member can be realized by virtue of its own elasticity or other expansion mechanisms, which is not limited in the present disclosure. In an exemplary embodiment, the expansion mechanism includes, for example, an eccentric cam driven by the applicator device so that the clamping member can be biased to the expanded position.

A retaining element may be disposed to hold the clamping member in the retracted state during insertion of the vascular interface assembly into the incision in the vascular wall. The retaining element is configured to surround the clamping member, for example, so as to limit the expansion of the clamping member. Furthermore, the clamping member can be released from its constraint through an operation of the applicator device. However, it should be understood that the retaining element is optional and may be omitted when the clamping member is expanded through an expansion mechanism.

In an advantageous embodiment, the retaining element can be integrally formed with the flange, so as to simplify the overall structure of the vascular interface assembly.

A synthetic vascular graft may be attached at the proximal side of the vascular interface assembly, so as to be bridged onto a target blood vessel through the vascular interface assembly. The synthetic vascular graft can be clamped by hemostatic forceps, and can be opened as needed, for example, when connecting it to another synthetic vascular graft or another blood vessel.

Thus, by utilizing the vascular anastomosis assembly of the present disclosure, vascular connection can be readily achieved. For example, a connection between a bypass vessel and a main vessel can be established to overcome conditions such as local occlusive lesions of the main vessel. The vascular anastomosis assembly can also be used to treat other conditions and achieve vascular bridging. Since the entire surgical procedure can be accomplished by operating the applicator device, the surgical process is simplified, the technical skill required for the physician or operator is reduced, the difficulty of implementation is low, the operation time is shortened, and the resulting surgical trauma is minimized. This facilitates reducing patient suffering and shortening recovery time.

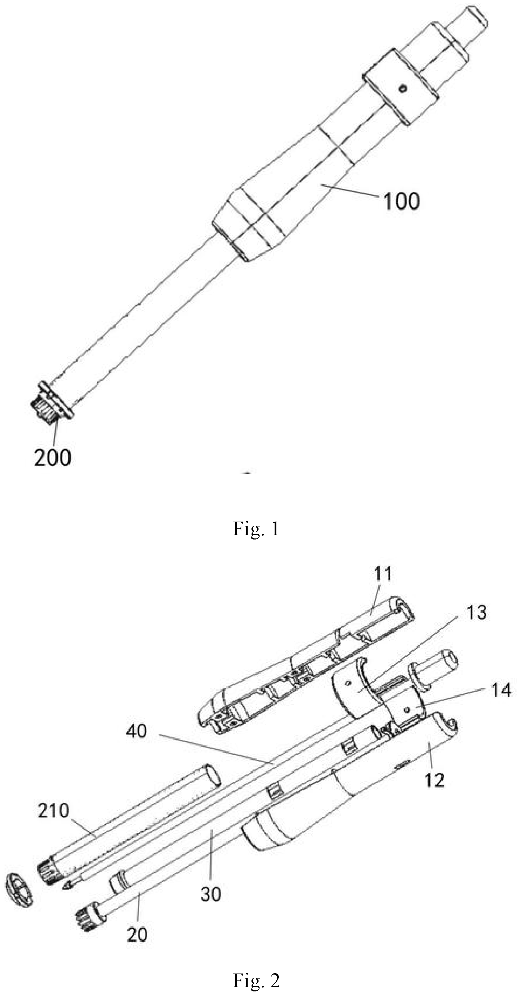

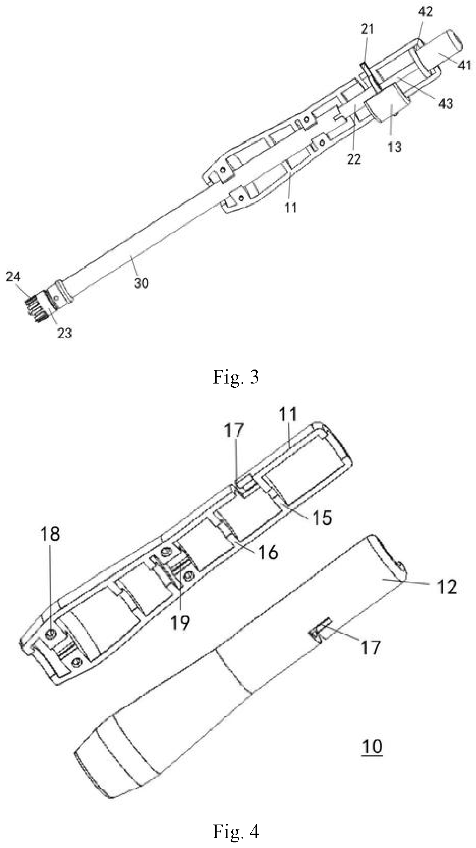

The vascular anastomosis assembly according to the present disclosure will be described in details below with reference to the accompanying drawings. In the accompanying drawings, Fig. 1 illustrates a perspective view of a vascular anastomosis assembly according to the present disclosure, Fig. 2 is an exploded perspective view illustrating the vascular anastomosis assembly shown in Fig. 1, Fig. 3 is a partial cross-sectional view of an applicator device 100 with a vascular interface assembly 200 removed, and Fig. 4 is an exploded perspective view of a handle10.

As shown in Figs. 1 and 2, a vascular anastomosis assembly 1 according to the present disclosure includes an applicator device 100 and a vascular interface assembly 200. The vascular interface assembly 200 is releasably engaged onto the applicator device 100, so as to be placed into an incision in the vascular wall through the applicator device 100. As shown in Fig. 2, the applicator device 100 includes: a handle 10, through which a surgeon or an operator can operate the vascular anastomosis assembly 1; a sheath 30 extending from a distal end of the handle 10; a cutting assembly 20 slidably accommodated in an internal space formed by the handle 10 and the sheath 30; and a piercing assembly 40 slidably accommodated in the internal space.

As shown in more details in Fig. 4, the handle 10 is generally composed of two halves 11 and 12. The two handle halves 11 and 12 are formed from a polymer material, for example, by injection molding, 3D printing or the like. Handle halves 11 and 12 are substantially mirror images of each other; therefore, only the housing half 11 is described in details. The interior of the housing half 11 includes a plurality of partition walls 15, 16 to increase the strength of the housing while forming the positioning portions for the sheath 30, the cutting assembly 20, and the piercing assembly 40. In addition, each handle half 11 and 12 is further formed with a mounting hole 18 for assembling the two handle halves 11 and 12 together, for example, by screws or the like. However, the present disclosure is not limited thereto, and other assembling or fastening methods may be utilized, such as adhesive bonding, welding, etc. Moreover, although not shown in the drawings, the handle 10 may be provided with a mark to indicate or signify an operating position or direction.

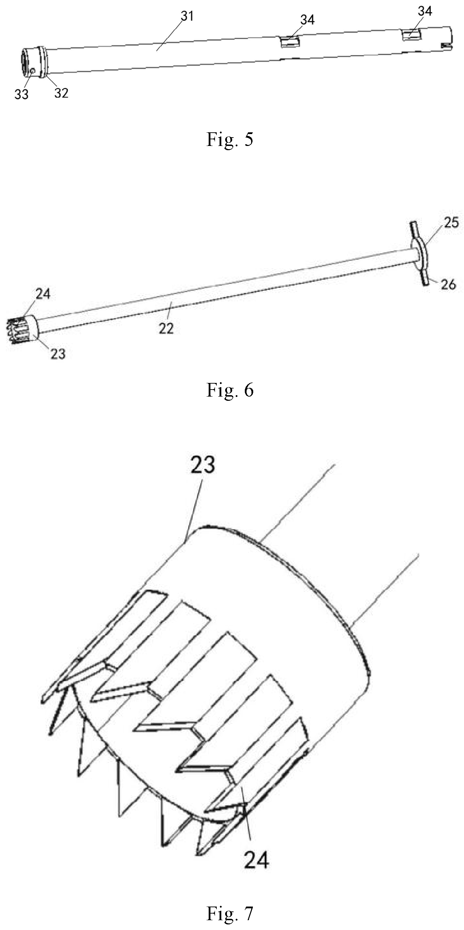

As shown in Figs. 3 and 4, in combination with Fig. 5 which illustrates a perspective view of the sheath 30, the sheath 30 is formed from a metal, for example, and includes an elongated main body 31. At least one protrusion 33 is formed along a circumferential direction of the distal end of the elongated main body 31, and the protrusion 33 is releasably engaged with the vascular interface assembly 200 described below. A shoulder 32 is further formed at a proximal side of the protrusion 33. The shoulder 32 serves as, for example, a mounting and positioning portion for the vascular interface assembly 200 described below, and can push the vascular interface assembly forward during implantation. In addition, a plurality of notches 34 are formed on the main body 31. These notches 34 are engaged with buckles 19 disposed inside the handle halve 11, thus fixing the sheath 30 onto the handle 10. Although not shown, the main body 31 of the sheath 30 is hollow, so as to accommodate the cutting assembly and the piercing assembly, as will be described in details below.

As shown in Fig. 6, which illustrates a cutting assembly 20, the cutting assembly 20 includes a hollow rod portion 22, a cutting head 24 is disposed at a distal end of the hollow rod portion 22, and a baffle 21 is disposed at a proximal end of the hollow rod portion 22. The baffle 21 is detachably fixed onto the proximal end of the rod portion 22, and includes a stopper portion 25 and at least one arm 26 extending from the stopper portion 25, the stopper portion 25 has a dimeter greater than that of the hollow rod portion 22. Although Fig. 6 shows that the baffle 21 includes two arms 26 extending oppositely, the present disclosure is not limited thereto, and the baffle 21 may include a plurality of arms or even a single arm. Referring to Fig. 3 again, the rod portion 22 is slidably accommodated in the hollow main body 31 of the sheath 30, with the cutting head 24 protruding beyond the distal end of the hollow main body 31. In addition, the arm 26 of the baffle 21 protrudes out of the handle 10 through a slit 17 formed on the handle 10 and is engaged with an operating ring 13. The operating ring 13 is slidably sleeved over an outer periphery of the handle 10, whereby a longitudinal movement of the cutting assembly 20 inside the handle 10 can be controlled by moving the operating ring 13 longitudinally along the outer periphery of the handle 10. A biasing element (not shown), for example, a coil spring is further provided, with one end of the coil spring abutting against a partition plate 16 inside the handle 10 and the other end of the coil spring abutting against the stopper portion 25 of the baffle 21, so that the cutting assembly 20 is biased proximally, i.e., biased toward the retracted position. As shown in Fig. 1, in the retracted position, the cutting head is retracted into the vascular interface assembly 200.

The cutting head 24 may be integrally formed with the rod portion 22, or may be separately formed and then fixed onto the rod portion 22 by welding, riveting, or the like. As another alternative, the cutting head 24 may be detachably connected to the rod portion 22. Such a detachable connection may be achieved, for example, by threaded connection, snap-fitted connection, pin, snap ring, or the like. Thus, when a blade of the cutting head 24 becomes less sharp after repeated use, the cutting head 24 can be replaced. Alternatively, different types of blades may be provided to satisfy different cutting requirements.

As shown in Fig. 4, the slit 17 on the handle is formed into L shape, including a longitudinally extending portion and a circumferentially extending portion. Thus, by passing the arm 26 of the baffle 21 through the slit 17, when the cutting assembly is manipulated via the operating ring 13, the slit 17 guides a longitudinal movement of the cutting assembly; and when the arm 26 moves into the circumferentially extending portion of the slit, the slit 17 allows the operating ring 13 to rotate the cutting assembly. The circumferentially extending portion ranges, for example, from 60° to 120°, thereby allowing the cutting head 24 at the distal end of the cutting assembly to rotate by a specific angle to perform the cutting operation. As shown in Fig. 4, the circumferentially extending portion of the slit 17 may be formed to extend clockwise from the longitudinally extending portion, thereby allowing the cutting head 24 to rotate clockwise. However, the present application is not limited thereto. Instead, through a combination of the circumferentially extending portion and the longitudinally extending portion, the cutting head may be allowed to rotate clockwise, counterclockwise, or in both directions to perform cutting. Preferably, a mark (not shown) is provided on the handle to indicate the rotation angle of the cutting head 24.

As shown in Fig. 3 and Figs. 6 to 9, the cutting head may include a cylindrical main body 23 and a plurality of blades 24 fixed onto the main body 23 by means of, for example, welding, riveting, adhesive bonding or the like. Compared to the technical solution in which the main body 23 and the blades 24 are integrally formed, forming the blades first and then fixing the blades onto the main body 23 can simplify the manufacturing process of the blades and facilitate the formation of the cutting edges of the blades.

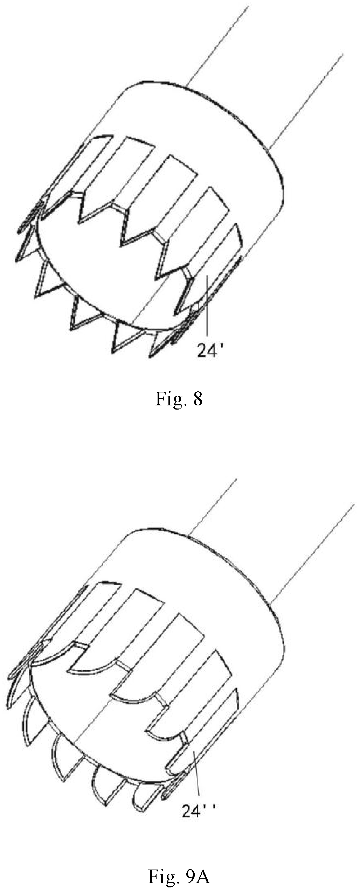

As shown in Figs. 7 to 9A, which illustrate three optional forms of the blades. As shown in Fig. 7, the blade 24 is formed with a cutting edge that extends obliquely along one direction. With such a cutting edge, the cutting head is allowed to rotate in only one direction to perform cutting. Fig. 8 illustrates a blade 24' having a cutting edge that extends obliquely in two directions, which allows the cutting head to rotate in both of the two directions to achieve cutting. Fig. 9A illustrates a blade 24" with an arcuate cutting edge. This blade 24" is similar in shape to a surgical scalpel. Compared to the oblique cutting edges shown in Figs. 7 and 8, the arcuate cutting edge can generate greater tangential force and forward thrust during cutting, thus allowing tissues to be pierced more quickly. Compared to a traditional straight cutting edge, it allows the blade to pass through the tissue being cut more easily, thus reducing the operating force and improving the cutting efficiency. In addition to the arcuate cutting edge, such blade 24" may also be provided with a cutting edge at the other side to enhance the piercing effect; for example, it can easily puncture the blood vessel and then begin cutting. Furthermore, as described above, when the cutting head 22 is detachable, a variety of cutting heads equipped with different blades can be provided, thus allowing different cutting heads to be selected according to the surgical situation or the cutting sites.

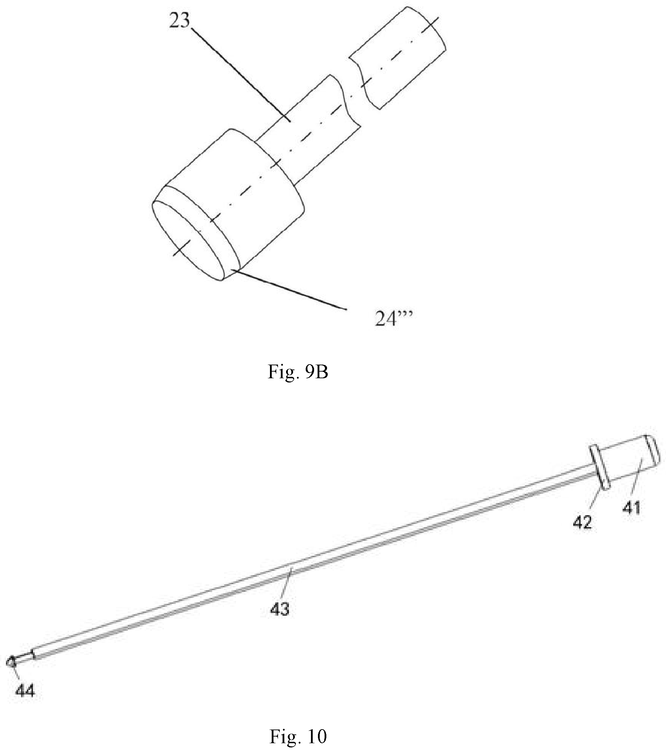

Although the above describes a structure in which the blade is formed separately and attached to the main body, as well as various forms of the blades, the present disclosure is not limited thereto. Fig. 9B illustrates another cutting head applicable in the present disclosure.

As shown in Fig. 9B, a cutting edge 24‴ may be formed at a distal edge of the cylindrical main body 23, thereby forming a tubular cutter configuration. The cutting edge may extend around the full circumference; however, the present disclosure is not limited thereto. A plurality of cutting-edge portions (not shown) may also be provided in the circumferential direction. These cutting-edge portions are regularly distributed around the circumference with gaps therebetween.

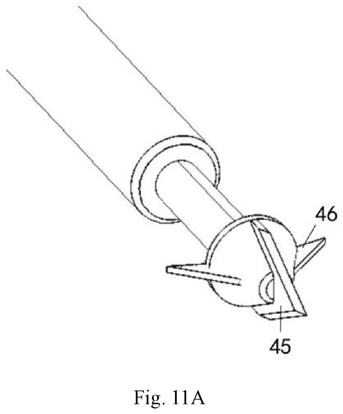

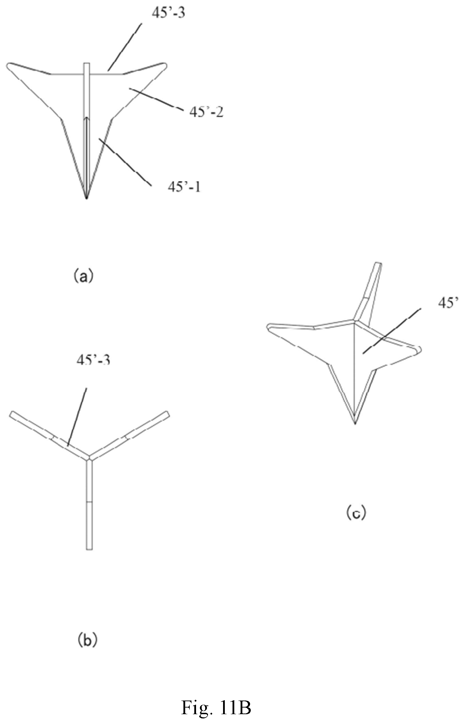

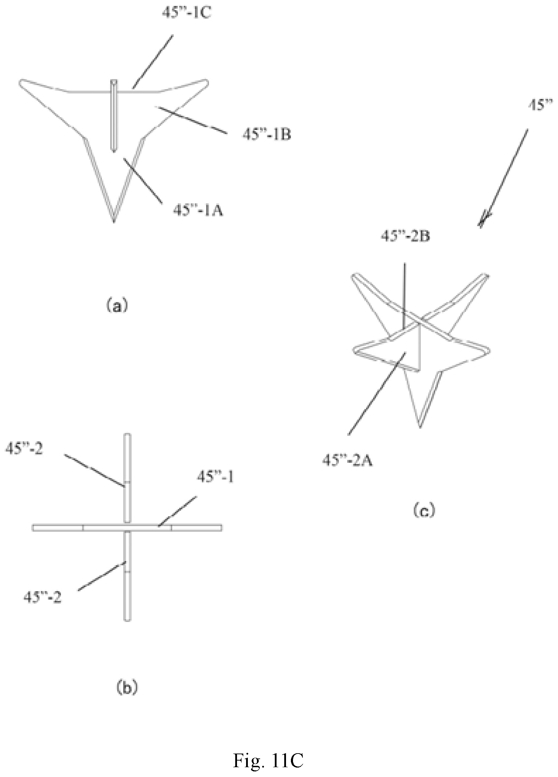

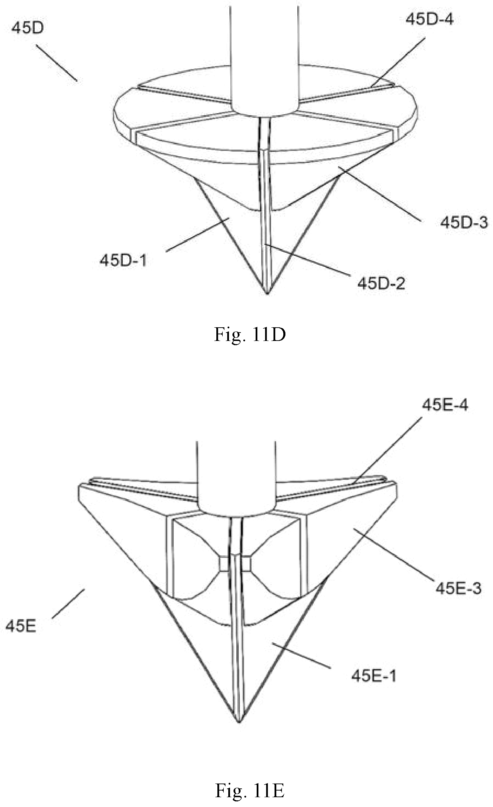

Fig. 10 illustrates a perspective view of the piercing assembly 40, and Fig. 11 illustrates an enlarged view of the distal end of the piercing assembly 40.

As shown in Fig. 10, the piercing assembly 40 includes a rod portion 43, a button 41 and a shoulder 42 disposed at the proximal end of the rod portion 43, and a sharp tip portion 44 disposed at the distal end of the rod portion 43. The rod portion 43 is inserted into the hollow rod portion 22 of the cutting assembly. As shown in Fig. 3, the button 41 protrudes from the proximal end of the handle, and the shoulder 42 is caught at the tip opening of the handle to prevent the button 41 from coming off. Additionally, although not shown, a biasing device such as a compression spring is disposed between the partition plate 15 of the handle and the shoulder 42 of the button 41 so that the piercing assembly 40 is biased toward its retracted position. In the retracted position, the tip portion 44 of the piercing assembly 40 is accommodated within the cutting head of the cutting assembly. Thus, by pressing the button 41, the tip portion 44 of the piercing assembly can be protruded beyond the distal end of the cutting assembly, thereby piercing the target vascular wall. As shown in Fig. 11A, the tip portion 44 includes a piercing blade 45 forming the tip, and a barb portion 46. When viewed along the longitudinal direction, the piercing blade 45 and the barb portion 46 generally form a cross shape; however, the present disclosure is not limited thereto, and other forms of piercing tips and barbs may be adopted. By means of the piercing tip and the barb, when the piercing tip passes through the vascular wall by pressing the button 41, and upon release of the pressure on the button 41, the piercing assembly moves toward the retracted position under the action of the spring, and can pull the vascular wall via the barb, as will be described later. Preferably, the button 41 is provided with a mark to indicate the degree or direction of depression of the button 41.

The piercing blade 45 may be formed separately and fixed onto the piercing tip by welding, adhesive bonding, or the like, thereby simplifying the manufacturing process. Additionally, the entire piercing tip may be fixed onto the rod portion 43, or may alternatively be detachably connected to the rod portion 43, for example, by threading, snap-fitting or the like, thereby allowing replacement of the piercing tip.

Figs. 11B and 11C illustrate two alternative embodiments of the piercing blade. Fig. 11B illustrate views of an embodiment of a three-piece piercing blade 45', wherein (a) shows a side view, (b) shows a top view, and (c) shows a perspective view. As shown in Fig. 11B, the piercing blade 45' is composed of three identical pieces and fixed onto the rod portion 43, with each piece including a piercing portion 45'-1 and a tail portion 45'-2. The piercing portion 45'-1 forms a cutting edge at its distal edge. The tail portion 45'-2 extends outward from the end of the piercing portion 45'-1 at an angle greater than 100 degrees, preferably greater than 120 degrees, and a recessed, substantially flat portion 45'-3 is formed at the proximal end of the tail portion 45'-2, whereby the tail portion is shaped similarly to an aircraft wing.

Fig. 11C illustrates views of an embodiment of a four-piece piercing blade 45", wherein (a) is a side view, (b) is a top view, and (c) is a perspective view. As can be seen from Fig. 11C, the piercing blade 45" includes a main blade portion 45"-1 and a positioning blade 45"-2. The main blade portion 45"-1 includes a piercing portion 45"-1A and symmetrically arranged tail portions 45"-1B. Similar to Fig. 11B, the tail portion 45"-1B also includes a recessed, substantially flat portion 45"-1C at its proximal end. The positioning blade 45"-2 has a substantially triangular shape, with its distal edge forming a cutting-edge portion 45"-2A and its proximal end forming a slightly recessed, flat portion 45"-2B. The positioning blades 45"-2 may be symmetrically arranged at both sides of the main blade portion 45"-1, thereby forming a cross shape in a top view, as shown in (b) of Fig. 11C.

Fig. 11D illustrates a piercing blade 45D according to another embodiment of the present application. The piercing blade 45D includes a generally inverted conical main body portion 45D-3. The main body portion 45D-3 is formed with slits 45D-4 perpendicular to one another. A main blade portion 45D-1 and a positioning blade 45D-2 are embedded in the slits 45D-4 and protrude from the slits 45D-4. By including the cylindrical main body portion 45D-3, when the piercing blade 45D penetrates the blood vessel and retracts, it can more reliably hold the vascular portion. Alternatively, the main body portion may also be formed with three slits arranged at 120° relative to each other, with a blade embedded in each slit, and all the three blades are identical with each other. A circular-shaped planar surface of the main body portion serves as a positioning surface (with larger area) to more reliably hold the vascular portion. Fig. 11E illustrates a piercing blade 45E according to yet another embodiment. In the piercing blade 45E shown in Fig. 11E, the inverted conical main body portion 45E-3 has three portions along the circumference cut away to form three corners, and a slit 45E-4 is formed at each of the three corners. Three substantially identical blades 45E-1 are embedded in the slits 45E-4, thereby reducing the overall size of the piercing blade 45E while achieving the effects similar to those of the piercing blade 45D.

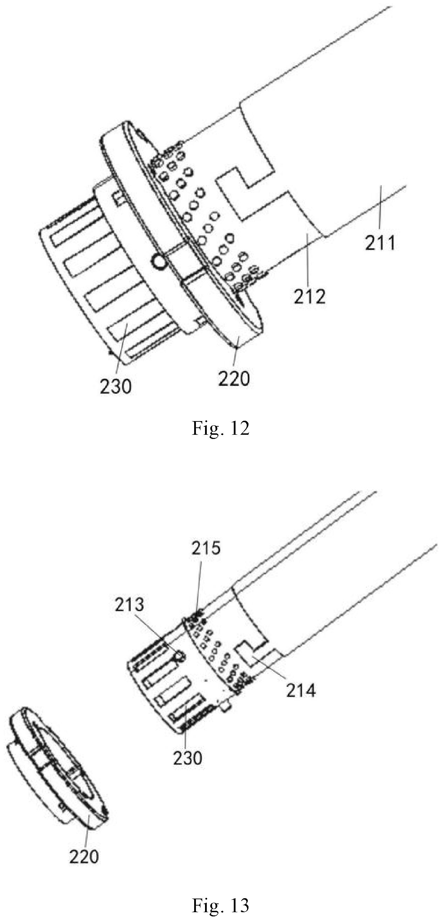

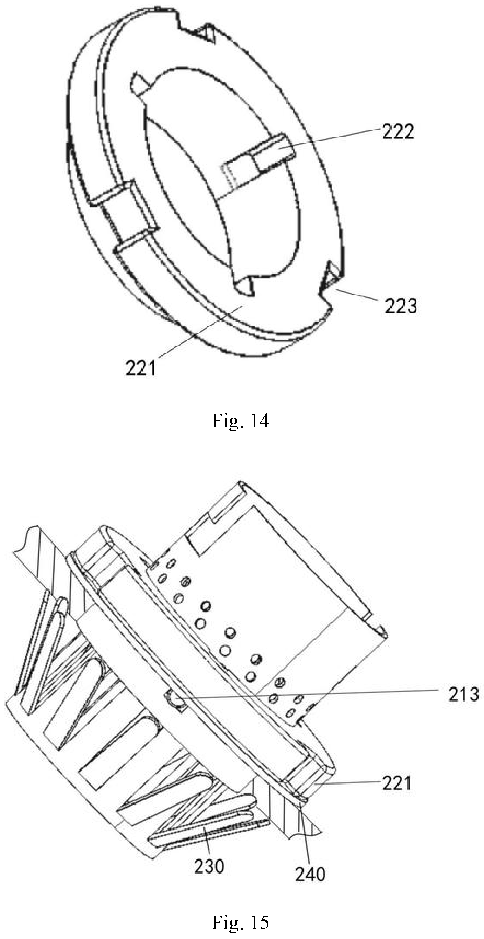

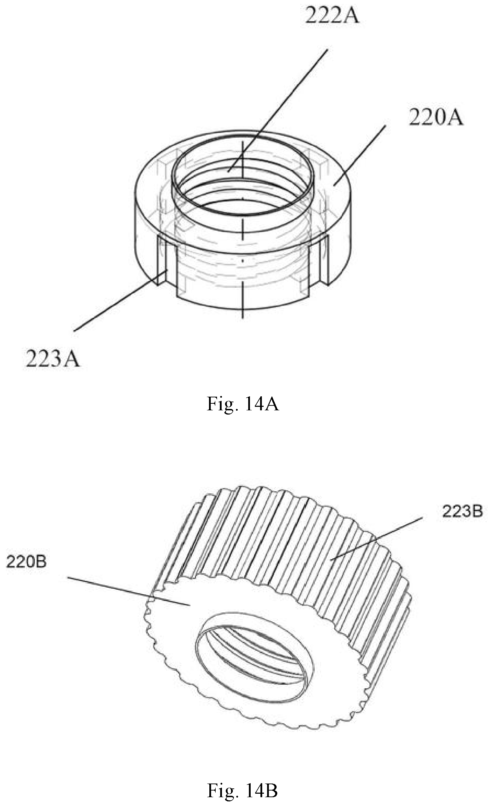

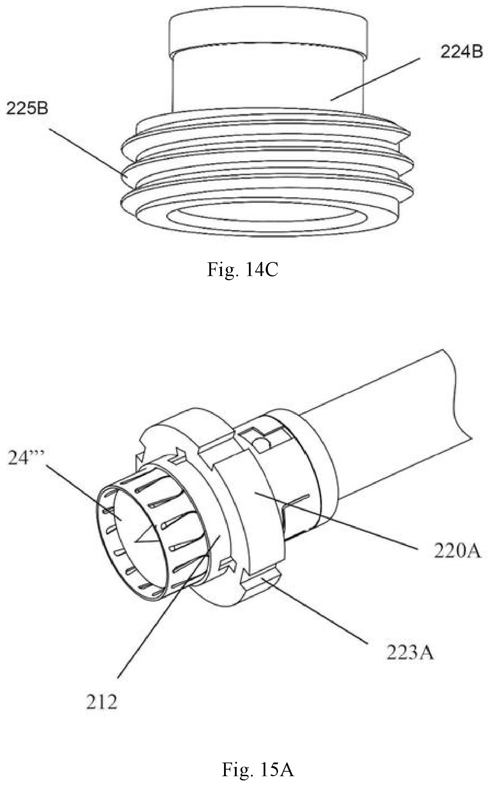

The structure of the vascular interface assembly 200 is described below with reference to Figs. 12 to 15. Fig. 12 illustrates a perspective view of the vascular interface assembly 200, with the clamping member in a retracted state; Fig. 13 illustrates an exploded view of the vascular interface assembly 200; Fig. 14 illustrates a perspective view of a triggering member 220; and Fig. 15 illustrates a schematic view of the clamping member in an expanded state. As shown in Figs. 12 and 13, the vascular interface assembly 200 includes, for example, a cylindrical main body portion 212, a synthetic vascular graft 211 connected to the proximal end of the main body portion 212, and a triggering member 220 sleeved over the outer periphery of the main body portion 212. The main body portion 212 may be formed by rolling and welding a thin-walled material or by direct machining of a tubular material, and has a diameter ranging from 5 mm to 20 mm. The thin-walled material, for example, is stainless steel, nickel-titanium alloy or other biocompatible materials, with a wall thickness ranging from 0.1 mm to 2 mm, e.g., 0.2 mm. A plurality of small holes 215 may be formed at the proximal portion of the main body portion 212 to facilitate connecting with the synthetic vascular graft 211, for example, by suturing, adhesive bonding or the like. In addition, a plurality of bumps 213 are formed, along a circumferential direction, at the middle of the main body portion 212, and these bumps 213 serve as a guiding and limiting device for the triggering member 220. A plurality of clamping members 230 are machined, along a circumferential direction, at the distal portion of the main body portion 212 by laser cutting or the like. The clamping member 230, for example, is subject to a heat treatment and setting process so as to be featured with elasticity, and hence can be expanded by virtue of its own elasticity.

An L-shaped slot 214 is formed at the proximal end of the main body portion 212. The slot 214 includes a longitudinally extending portion extending distally from a proximal edge of the main body portion 212, and a circumferentially extending portion extending circumferentially, by a certain length, from a distal end of the longitudinally extending portion. The slot 214 is engaged with the protrusion 33 of the sheath 30 (see Fig. 5). Thus, when the protrusion 33 is located within the circumferentially extending portion of the slot 214, the vascular interface assembly is connected to the applicator device. As the sheath 30 rotates relative to the vascular interface assembly, the protrusion 33 can be rotated into alignment with the longitudinally extending portion of the slot 214, thereby allowing the vascular interface assembly to be released from the applicator device. In this way, the protrusion and the L-shaped slot constitute a release mechanism. However, it should be understood that the present disclosure is not limited thereto, and may include other types of release mechanisms, such as a quick-release snap-fitting structure or the like. Release of the release mechanism does not necessarily have to be achieved by rotation of the applicator device, but may also be achieved by providing a dedicated release device.

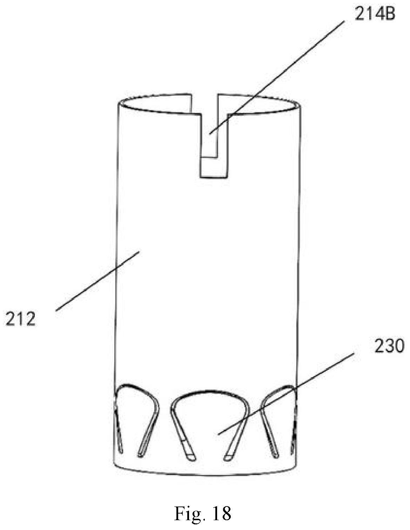

However, the slot 214 may also take other forms. For example, as shown in Fig. 18, the slot 214B is formed as a straight cutout, but the present disclosure is not limited thereto. Moreover, as shown in Fig. 18, the clamping member 230 may also be formed in a petal shape, so as to be better clamped and held on the inner wall of the blood vessel.

As shown in Figs. 12 and 14, the triggering member 220 is movable longitudinally around the main body portion 212, and includes a cylindrical portion and a flange portion 221 extending radially from the cylindrical portion. On the inner peripheral surface of the triggering member 220, a plurality of grooves 222 (three are shown in the figure) are formed at intervals, and the grooves 222 are respectively engaged with the bumps 213 on the main body portion 212. Thus, when the triggering member 220 moves along the main body portion 212, the bumps 213 guide the movement of the triggering member 220 and limit the range of movement of the triggering member 220, so as to prevent the triggering member 220 from disengaging from the main body portion 212. A plurality of notches (three are shown in the figure) are formed on the outer periphery of the flange portion 221. These notches are configured to be engaged with corresponding surgical instruments to adjust the position of the triggering member 220.

Thus, when the triggering member 220 is in a distal position, it surrounds the clamping member 230 to prevent the clamping member 230 from expanding by virtue of its own elastic force. As the triggering member 220 moves proximally, it clears away from the clamping member 230, thereby releasing the restraint on the clamping member 230 and allowing the clamping member 230 to expand by virtue of its own elastic force. Thus, when the vascular interface assembly 200 is placed into the incision in the vascular wall, the expanded clamping member 230 supports the inner side of the vascular wall. Since the outer diameter of the flange portion 221 is larger than the outer diameter of the main body portion 212, when the main body portion 212 is placed into the incision in the vascular wall, the flange portion 221 will be abutted against the outer side of the vascular wall. Meanwhile, since the inner diameter of the flange portion 221 is smaller than the outer diameter of the clamping member 230 after expansion, the flange portion 221 abutted against the outer side of the vascular wall and the expanded clamping member 230 can form fixation and sealing of the blood vessel, thus sandwiching the vascular wall between the clamping member 230 and the flange portion 221 (see Fig. 15). Blood within the blood vessel can then flow into the synthetic vascular graft through the hollow space in the main body portion 212 of the vascular interface assembly.

Although in the above embodiments, the engagement between the grooves 222 and the bumps 213 is described as enabling the triggering member 220 to translate along the main body portion 212, the present disclosure is not limited thereto, and threaded engagement may also be adopted, as shown in Fig. 14A. As shown in Fig. 14A, the triggering member 220A includes internal threads 222A, and correspondingly, external threads (not shown) or protrusions are formed on the outer periphery of the main body portion 212 to be engaged with the internal threads of the triggering member 220A. Notches 223A are formed on the outer periphery of the triggering member 220A. Thus, as shown in Fig. 15A, after assembled onto the main body portion 212, the triggering member 220A can be rotated and axially moved along the external threads on the outer periphery of the main body portion 212, by means of a tool snap-fitting with the notches 223A, to a position where the clamping member 230 is expanded. Subsequently, the triggering member 220A can be rotated in a reverse direction, causing the triggering member 220A to be pressed against the outer side of the blood vessel, thereby more effectively compressing the blood vessel tightly and preventing blood leakage.

Fig. 14B illustrates a further embodiment of the triggering member 220B. As shown in Fig. 14B, the outer periphery of the triggering member 220B is formed with outer ribs 223B to facilitate gripping the outer periphery of the triggering member 220B by a surgeon or assistance and rotating the triggering member 220B during a surgery.

Optionally, a threaded sleeve 224B may be fixedly connected to the outer periphery of the main body portion 212. As shown in Fig. 14C, the threaded sleeve 224B may be fixedly connected to the outer periphery of the main body portion 212 by welding or adhesive bonding, or the threaded sleeve 224B may be integrally formed with the main body portion 212. Threads 225B are formed on the outer periphery of the threaded sleeve 224B to be engaged with the internal threaded holes of the triggering member 220A or 220B. Thus, by rotating the triggering member 220A or 220B, the triggering member 220A or 220B can be moved along an axial direction of the main body portion 212.

The triggering member 220A or 220B may be moved away from the clamping member 230 by screwing on the threaded sleeve 224B, so as to release the clamping member 230. Furthermore, once the clamping member 230 is clamped and held in place, the triggering member 220A or 220B can be rotated in the reverse direction, so that the flange portion 221 of the triggering member 220A or 220B is pressed against the outer wall of the blood vessel. However, the present disclosure is not limited thereto. The triggering member and the flange portion may be separate components. That is, a press ring (not shown) may be further provided between the triggering member 220A or 220B and the clamping member. The press ring is substantially annular and moves axially along the outer periphery of the main body portion, in association with the triggering member 220A or 220B. Preferably, the press ring moves only axially without rotating, which is achieved, for example, by engagement between a slot in the press ring and a limiting pin on the outer periphery of the main body portion.

To further enhance the sealing between the vascular interface assembly and the vascular incision, optionally, a hemostatic member and/or a sealing member 240 may be disposed on a surface of the flange portion 221 facing the vascular wall. The sealing member includes, for example, a hemostatic felt, a gauze pad, or the like. In addition, the surface of the triggering member 220A facing the blood vessel may also be provided with a sealing material or a sealing member to further improve the sealing effect.

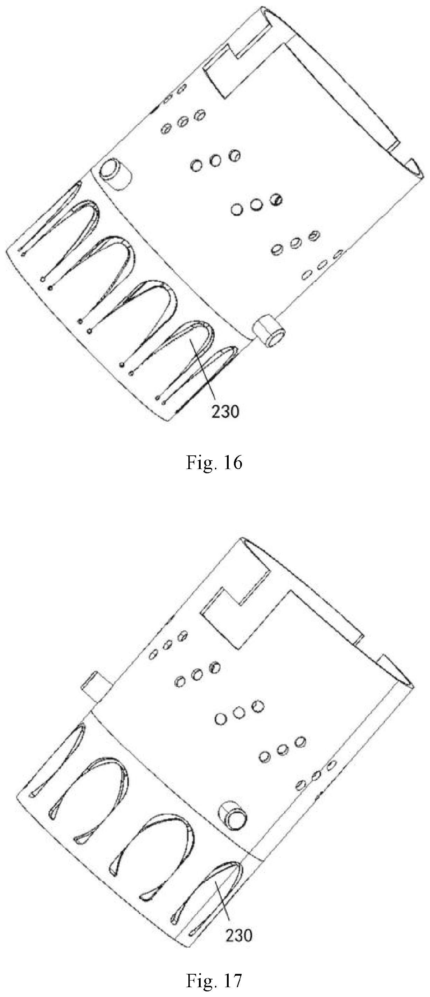

Figs. 16 and 17 illustrate two alternative embodiments of the clamping member 230. As shown in Figs. 16 and 17, the clamping member 230 may have a rounded free end, so as to reduce damage to the vascular wall when clamped and held thereon, and also prevent displacement or detachment from the blood vessel after implantation.

Although the foregoing describes the clamping member 230 as being integrally formed with the main body portion 212 and cut out from the main body portion 212, for example, by laser cutting or the like, the present disclosure is not limited thereto. Other forms of the clamping member 230 may be adopted. For example, the clamping member may be formed separately and connected to the main body portion, such as by welding. The clamping member 230 may be made of a material such as a shape memory alloy and may thereby be expanded to the expanded state by virtue of its own elasticity. However, the present disclosure is not limited thereto. Other ways of expanding the clamping member 230 to the expanded state may also be adopted, such as an eccentric cam, high-pressure gas or liquid actuation, etc. In such cases, the triggering member may be a mechanism or element that applies or actuates the eccentric cam, high-pressure gas or liquid, or the like.

In the description above, the flange portion 221 is provided on the triggering member 220. The flange portion 221 may be integrally formed with the triggering member 220, or the flange portion 221 and the triggering member 220 may be formed separately and then joined together by welding, adhesive bonding, or the like. However, the present disclosure is not limited thereto. The flange portion does not necessarily have to be formed together with the triggering member. The flange portion 221 may be formed separately on the main body portion 212, while the triggering member 220 is separately sleeved over the main body portion 212 and driven, for example, by a driving mechanism such as a pull cord, a push rod, or the like.

The mode of operation of the vascular anastomosis assembly of the present disclosure will be briefly described below with reference to Fig. 1:
Step 1: Preparing the vascular anastomosis assembly. In an initial state, the vascular interface assembly 200 is connected to the applicator device 100, and the synthetic vascular graft is sleeved outside the sheath 30. In this state, the triggering member 220 is at its distal position, thereby surrounding the clamping member 230 to prevent the clamping member from expanding.

Step 2: Aligning the vascular anastomosis assembly with a target placement position on the blood vessel. Preferably, the blood vessel is stretched to facilitate penetration and cutting by the piercing assembly and the cutting assembly.

Step 3: Abutting the distal end of the vascular anastomosis assembly against the vascular wall, and pressing the button 41, thereby moving the piercing assembly to the extended position. In the extended position, the piercing tip of the piercing assembly pierces into and penetrates through the vascular wall.

Step 4: Releasing the button 41, then moving the operating ring 13 downward along the outer periphery of the handle to move the cutting head 23 of the cutting assembly to the extended position. In the extended position, the cutting blade 24 of the cutting head 23 pierces into the vascular wall. Upon reaching the extended position, the operating ring 13 can rotate the cutting assembly, thereby cutting out an incision in the vascular wall with the cutting blade 24. In the case of adopting a tubular cutter as shown in Fig. 9B, rotating the cutting assembly is not necessary; instead, the incision is cut out directly, thereby improving the cutting effect and the shape integrity of the incision.

Step 5: after cutting out the incision, pulling an excised portion of the vascular wall out of the incision via the barbs or tail portions on the piercing tip, collecting the excised portion within the cutting head 23, and simultaneously inserting the distal portion of the vascular interface assembly into the incision. By utilizing the wing-shaped tail portions as shown in Figs. 11B and 11C, the positioning effect of the piercing assembly on the blood vessel can be improved. In this way, it's easier and more efficient to form the incision in the blood vessel through the cooperation of the piercing assembly and the cutting assembly.

Step 6: Continuing to insert the vascular interface assembly while abutting the flange portion 221 of the triggering member 220 of the vascular interface assembly against the outer side of the vascular wall at the incision. As the main body portion of the vascular interface assembly is further inserted inward, the triggering member 220 slides on the main body portion of the vascular interface assembly because the flange portion 221 is blocked by the vascular wall. Thus, the triggering member 220 moves to its proximal position and releases the blocking action on the clamping member 230. The clamping member 230 is expanded to the expanded position under its own elastic force, thereby sandwiching the vascular wall between the clamping member 230 and the flange portion 221. With the assistance of the clamping member 230 and the flange portion 221, and further with the aid of the sealing member provided on the flange portion 221, the incision in the blood vessel is sealed, which enables communication between the blood vessel and the synthetic vascular graft. In the case of the triggering member 220A, an external tool may be used to rotate the triggering member 220A so that the triggering member 220A releases the blocking action on the clamping member 230, to allow the clamping member 230 to be expanded. After the clamping member 230 is expanded, the triggering member 220A is rotated in the reverse direction so that the triggering member 220A is abutted against the outer wall of the blood vessel, thereby improving the vascular sealing effect.

Step 7: Rotating the handle after the vascular interface assembly is in place, so that the protrusion 33 on the sheath 30 disengages from the L-shaped slot 214 in the main body portion of the vascular interface assembly, thereby releasing the vascular interface assembly and withdrawing the applicator device. Thus, the implantation of the vascular interface assembly is completed.

Generally, the synthetic vascular graft can be clamped with hemostatic forceps to prevent blood outflow. Subsequently, the synthetic vascular graft can be connected to a target device as needed to perform corresponding treatment. For example, in a bypass grafting procedure, the vascular anastomosis assembly of the present disclosure can be utilized to implant a vascular interface assembly at each of two locations on either side of a necrotic or occluded portion of the blood vessel. The synthetic vascular grafts are then connected to the respective blood vessels, thereby bypassing the necrotic or occluded portion.

After implanting the vascular interface assembly, the applicator device can be re-sterilized and fitted with a new vascular interface assembly for performing a new surgical procedure. Thus, costs are saved.

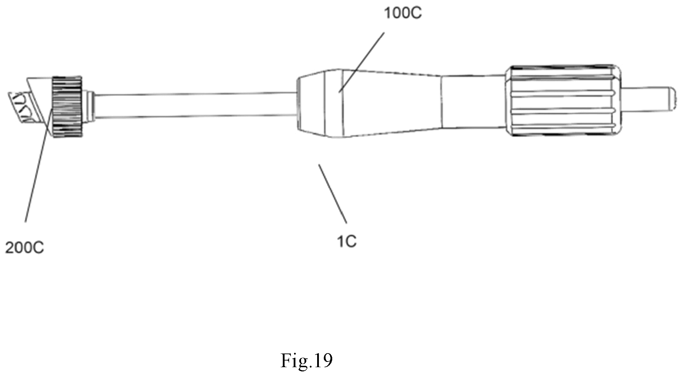

Although the foregoing describes embodiments in which the vascular anastomosis assembly is inserted and implanted substantially perpendicular to the vascular wall with reference to Figs. 1 to 18, the present disclosure is not limited thereto. With reference to Figs. 19 to 22 in conjunction with Figs. 1 to 18, an obliquely inserted vascular anastomosis assembly will be briefly described below.

Fig. 19 illustrates an overall view of the obliquely inserted vascular anastomosis assembly 1C. As shown in Fig. 19, the obliquely inserted vascular anastomosis assembly 1C differs from the above-described perpendicularly inserted vascular anastomosis assembly 1 only in the vascular interface assembly; the applicator devices of the two assemblies are the same. Therefore, the structure of the vascular interface assembly will be primarily described below.

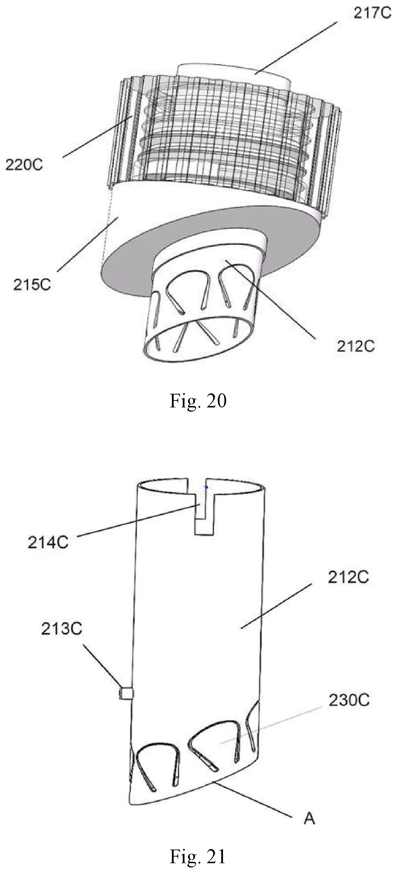

Fig. 20 illustrates a schematic view of the vascular interface assembly 200C. As shown in Figs. 20 to 23, the vascular interface assembly includes a main body portion 212C. As shown in Fig. 21, the main body portion 212C is substantially cylindrical, and differs from the main body portion 212B shown in Fig. 18 in that its distal end is formed as an inclined surface A. Similarly, the main body portion 212C includes, for example, a petal-shaped clamping member 230C and a slot 214C formed at the proximal end.

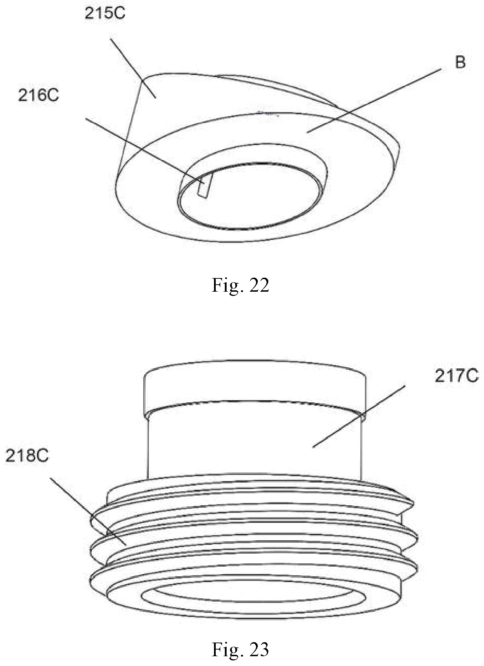

A limiting pin 213C is disposed on the outer periphery of the main body portion 212C. A press ring 215C, as shown in Fig. 22, is sleeved over the outer periphery of the main body portion 212C and is movable along the axial direction of the main body portion 212C. A limiting groove 216C is disposed on the inner periphery of the press ring 215C. The limiting groove 216C is engaged with the limiting pin 213C on the main body portion 212C to limit the range of axial movement of the press ring 215C along the main body portion 212C and to allow the press ring 215C to move only axially without rotating. Prior to implantation, the press ring 215C is sleeved outside the clamping member 230C, thereby limiting the expansion of the clamping member 230C. The press ring 215C is similarly provided with an inclined surface B at its distal end. The inclination angle of the inclined surface B may be the same as that of the inclined surface A, for example, the inclination angle is in the range of 20° to 70°, preferably in the range of 60° to 70°.

A threaded sleeve 217C is further fixed onto the outer periphery of the main body portion 212C. For example, the threaded sleeve 217C may be fixed onto the outer periphery of the main body portion 212C by welding or adhesive bonding. As shown in Fig. 23, the threaded sleeve 217C is fixed onto the outer periphery of the main body portion 212C, and external threads 218C are formed on the outer periphery of the threaded sleeve 217C. The triggering member 220C is similar to the triggering member 220B shown in Fig. 14B, i.e., the triggering member 220C is threadedly connected to the outer periphery of the threaded sleeve 217C and moving axially along the threaded sleeve (the main body portion) as the threaded sleeve rotates. Moreover, the rotation of the triggering member 220C simultaneously drives the press ring 215C to move axially without rotating.

When implanting the vascular interface assembly, the entire vascular anastomosis assembly can be inserted obliquely into the blood vessel, and the cutting head is used to cut out an opening in the vascular wall, while simultaneously inserting the main body portion 212C into the opening formed in the vascular wall. As the triggering member 220C is rotated, the press ring 215C is driven to move proximally along with the movement of the triggering member 220C, thereby releasing the clamping member 230. After the clamping member 230 is released and clamped against the inner wall of the blood vessel, the triggering member 220C is rotated again in the reverse direction, thereby driving the press ring 215C to be pressed against the outer wall of the blood vessel. In this way, the vascular interface assembly is pressed against the vascular wall, thus completing the surgical procedure.

As can be seen from the above description, the vascular anastomosis assembly of the present disclosure allows the vascular interface assembly to be implanted into the incision in the vascular wall simply through operations on the handle. The entire process does not require incision cutting, suturing, or similar procedures. Therefore, the vascular anastomosis assembly provided by the present disclosure can be used not only under open-chest conditions but also in minimally invasive interventional procedures, thereby simplifying the surgical process. In addition, the materials of the various components described above may be medical-grade materials or biocompatible materials, and the present disclosure is not limited in this regard. Furthermore, in order to prevent accidental trauma, the various components may be appropriately chamfered to avoid unnecessary sharp edges.

Although the above description employs a button to operate the piercing assembly and employs an operating ring to operate the cutting assembly, the present disclosure is not limited thereto, and other structures may be adopted. Furthermore, the present disclosure is not limited to manual operation of the above-described assemblies; instead, electrically, pneumatically, or hydraulically actuated structures may also be used. For example, a motor may be included in the handle to drive the piercing assembly or the cutting assembly to switch between the retracted position and the extended position.

Although the above description and drawings illustrate the counts of a plurality of clamping members, a plurality of shoulders, or a plurality of protrusions, it should be understood that these are exemplary, and a person skilled in the art may increase or decrease the counts as needed without impairing the effects or purposes of the present disclosure.

Accordingly, the following technical solutions are provided in accordance with the present disclosure:
Solution 1. A vascular anastomosis assembly, including:
   an applicator device, the applicator device including:
   a handle;
   a hollow sheath extending from the handle;
   a piercing assembly movably disposed within the sheath of the handle, the piercing assembly including a piercing member configured to pierce a vascular wall, and the piercing member being movable between a retracted position and an extended position;
   a cutting assembly movably disposed within the sheath of the handle, the cutting assembly being movable between a retracted position and a cutting position; wherein in the extended position, the cutting assembly is rotatable, by a predetermined angle, relative to the handle to cut out an incision in the vascular wall; and
   a vascular interface assembly releasably disposed at a distal end of the applicator device, and the vascular interface assembly including:
   a main body portion;
   a synthetic vascular graft extending from a proximal end of the main body portion;
   a clamping member disposed at a distal end of the main body portion, the clamping member being triggerable to an expanded position;
   a flange disposed on an outer periphery of the main body portion; and
   a triggering member, the triggering member being configured to trigger the clamping member to the expanded position such that the vascular wall is sealingly sandwiched between the clamping member and the flange.
Solution 2. The vascular anastomosis assembly according to Solution 1, wherein the applicator device further includes a first biasing device that biases the piercing assembly toward its retracted position.
Solution 3. The vascular anastomosis assembly according to Solution 1 or 2, wherein the applicator device further includes a second biasing device that biases the cutting assembly toward its retracted position.
Solution 4. The vascular anastomosis assembly according to any one of Solutions 1 to 3, wherein the cutting assembly includes a hollow rod accommodated within the hollow sheath of the handle, and a cutting head fixed onto a distal end of the hollow rod; the cutting head is cylindrical, and a plurality of blades are disposed at uniform intervals along a circumferential direction on a distal end of the cylindrical cutting head.
Solution 5. The vascular anastomosis assembly according to Solution 4, wherein the blade includes a cutting edge that is inclined along one direction.
Solution 6. The vascular anastomosis assembly according to Solution 4, wherein the blade includes a cutting edge that is inclined along two directions.
Solution 7. The vascular anastomosis assembly according to Solution 4, wherein the blade includes an arcuate cutting edge.
Solution 8. The vascular anastomosis assembly according to any one of Solutions 4 to 7, wherein the blade is welded to the distal end of the cutting head.
Solution 9. The vascular anastomosis assembly according to Solution 4, wherein a diameter of the cutting head ranges from 5 mm to 20 mm.
Solution 10. The vascular anastomosis assembly according to Solution 4, wherein a distal edge of the cutting head forms a cutting rim, thereby constituting a tubular cutter, and the cutting rim is preferably formed along an entire circumference of the distal edge.
Solution 11. The vascular anastomosis assembly according to any one of Solutions 4 to 10, wherein the cutting head is disposed within the main body portion of the vascular interface assembly, and an outer diameter of the cutting head is substantially equal to an inner diameter of the main body portion.
Solution 12. The vascular anastomosis assembly according to Solution 4 or 5, wherein the piercing assembly includes a rod portion accommodated within the hollow rod of the cutting assembly; the piercing member is fixed onto a distal end of the rod portion, and the piercing member includes a blade forming a sharp distal end and a barb extending proximally.
Solution 13. The vascular anastomosis assembly according to Solution 4 or 5, wherein the blade of the piercing assembly may be a three-piece blade or a four-piece blade. Each piece of the three-piece blade includes a cutting edge formed at a distal edge and a recessed, substantially flat portion formed at a proximal end. Each piece of the four-piece blade includes a main blade portion and positioning blade portions symmetrically arranged at both sides of the main blade portion, and each of the positioning blade portions includes a cutting edge at a distal edge and a recessed, substantially flat portion disposed at a proximal end.
Solution 14. The vascular anastomosis assembly according to any one of Solutions 1 to 13, further including a release mechanism which is actuatable to release the vascular interface assembly from the applicator device.
Solution 15. The vascular anastomosis assembly according to Solution 14, wherein the release mechanism includes a protrusion formed on a circumference of a distal end of the rod portion and a slot formed within a circumferential portion of the main body portion of the vascular interface assembly, and the protrusion is engaged in the slot.
Solution 16. The vascular anastomosis assembly according to Solution 15, wherein the slot is L-shaped and includes a circumferential portion extending along a circumferential direction and a longitudinal portion extending from the circumferential portion to a proximal edge of the main body portion. The protrusion is engaged in the slot such that a rotation of the handle enables the protrusion to move from the circumferential portion to the longitudinal portion, thereby releasing the vascular interface assembly from the applicator device.
Solution 17. The vascular anastomosis assembly according to any one of Solutions 1 to 16, wherein the clamping member is integrally formed with the main body portion, and the clamping member is placed in the expanded position by virtue of its own elasticity.
Solution 18. The vascular anastomosis assembly according to Solution 17, wherein the clamping member includes a plurality of claws arranged at intervals along a circumference of the main body portion, and the plurality of claws each have an elongated shape.
Solution 19. The vascular anastomosis assembly according to Solution 17, wherein the clamping member includes a plurality of claws arranged at intervals along a circumference of the main body portion, and the plurality of claws each have a rounded tip.
Solution 20. The vascular anastomosis assembly according to any one of Solutions 1 to 19, wherein the triggering member is arranged around the main body portion and is longitudinally movable, from a first position to a second position, along the main body portion. In the first position, the triggering member surrounds the clamping member to prevent the clamping member from being in the expanded position; and in the second position, the triggering member releases its restraint on the clamping member.
Solution 21. The vascular anastomosis assembly according to Solution 20, wherein one of the triggering member and the main body portion is provided with a groove, and the other one of the triggering member and the main body portion is provided with a bump; the groove is engaged with the bump to enable movement of the triggering member along the main body portion.
Solution 22. The vascular anastomosis assembly according to Solution 20, wherein the triggering member is provided with an internal thread and the main body portion is provided with an external thread; the internal thread of the triggering member meshes with the external thread of the main body portion so that the triggering member rotates and moves around the main body portion.
Solution 23. The vascular anastomosis assembly according to any one of Solutions 20 to 22, wherein a limiting member is disposed on the main body portion to restrict a movement range of the triggering member.
Solution 24. The vascular anastomosis assembly according to Solution 23, wherein the limiting member includes a bump disposed on a circumference of the main body portion.
Solution 25. The vascular anastomosis assembly according to any one of Solutions 1 to 24, wherein the flange is formed integrally with the triggering member.
Solution 26. The vascular anastomosis assembly according to any one of Solutions 1 to 25, wherein the handle includes a button connected to the piercing assembly to overcome the first biasing device and place the piercing assembly in the extended position.
Solution 27. The vascular anastomosis assembly according to any one of Solutions 1 to 26, wherein the handle includes an operating ring disposed on its outer periphery; the operating ring is connected to the hollow rod of the cutting assembly to overcome the second biasing device, place the cutting assembly in the extended position and rotate the cutting assembly.
Solution 28. The vascular anastomosis assembly according to any one of Solutions 1 to 27, wherein a hemostatic sealing member is disposed on a distal end face of the flange.
Solution 29. A vascular interface assembly configured to be sealingly disposed within an incision of a blood vessel, including:
   a cylindrical main body portion, an outer diameter of the main body portion being substantially equal to a diameter of the incision;
   a synthetic vascular graft extending from a proximal end of the main body portion;
   a clamping member disposed at a distal end of the main body portion, the clamping member being switchable between a retracted position and an expanded position;
   a flange disposed at a proximal end of the main body portion, wherein when the clamping member is in the expanded position, the vascular wall is sandwiched between the flange and the clamping member; and
   a triggering member, the triggering member being triggerable to switch the clamping member to the expanded position.
Solution 30. The vascular interface assembly according to Solution 29, wherein the clamping member switches from the retracted position to the expanded position by virtue of its own elasticity.
Solution 31. The vascular interface assembly according to Solution 29 or 30, wherein the triggering member is movable, between a first position and a second position, along the main body portion. In the first position, the triggering member surrounds the clamping member to hold the clamping member in the retracted position; and in the second position, the triggering member moves away from the clamping member and allows the clamping member to switch to the expanded position by virtue of an elasticity of the clamping member.
Solution 32. The vascular interface assembly according to any one of Solutions 29 to 31, wherein the triggering member and the flange are integrally formed.
Solution 33. The vascular interface assembly according to any one of Solutions 29 to 32, wherein a hemostatic sealing member is disposed on a surface of the flange facing the clamping member.
Solution 34. The vascular interface assembly according to any one of Solutions 29 to 33, wherein a limiting member is disposed on an outer periphery of the main body portion to restrict a movement range of the triggering member.
Solution 35. The vascular interface assembly according to Solution 34, wherein the limiting member is a protrusion formed on the outer periphery of the main body portion.

From the foregoing description, it will be apparent to those skilled in the art that while the methods and apparatus described herein constitute exemplary embodiments of the present disclosure, the present disclosure is not limited to these specific embodiments, and modifications may be made to such embodiments without departing from the scope of the present disclosure as defined by the claims. In addition, it should be understood that the present disclosure is defined by the claims, and this does not imply that any limitations or elements described in the exemplary embodiments set forth herein shall be incorporated into the interpretation of any claim element unless such limitations or elements are explicitly recited. Likewise, it should be understood that not all mentioned advantages or purposes of the present disclosure disclosed herein need to be satisfied in order to fall within the scope of any claim, because the present disclosure is defined by the claims and because even if not explicitly discussed herein, there may exist inherent and/or unforeseeable advantages of the claimed invention.

## Claims

1. A vascular anastomosis assembly, comprising:
an applicator device, the applicator device comprising:
a handle;
a hollow sheath extending from the handle;
a piercing assembly movably disposed within the sheath of the handle, the piercing assembly comprising a piercing member configured to pierce a vascular wall, the piercing member being movable between a retracted position and an extended position;
a cutting assembly movably disposed within the sheath of the handle, the cutting assembly being movable between a retracted position and a cutting position, wherein in the extended position, the cutting assembly is rotatable by a predetermined angle relative to the handle, so as to cut out an incision in the vascular wall; and
a vascular interface assembly, the vascular interface assembly being releasably disposed at a distal end of the applicator device, and the vascular interface assembly comprising:
a main body portion;
a synthetic vascular graft extending from a proximal end of the main body portion;
a clamping member disposed at a distal end of the main body portion, the clamping member being triggerable to an expanded position;
a flange disposed on an outer periphery of the main body portion; and
a triggering member, configured to trigger the clamping member to the expanded position such that the vascular wall is sealingly sandwiched between the clamping member and the flange.

2. The vascular anastomosis assembly according to claim 1, wherein the applicator device further comprises at least one of a first biasing device and a second biasing device, wherein
the first biasing device is configured to bias the piercing assembly to the retracted position of the piercing assembly, and the second biasing device is configured to bias the cutting assembly to the retracted position of the cutting assembly.

3. The vascular anastomosis assembly according to claim 1 or 2, wherein the cutting assembly comprises a hollow rod accommodated within the hollow sheath of the handle and a cutting head fixed at a distal end of the hollow rod, wherein
the cutting head is cylindrical and provided with a plurality of blades arranged at uniform intervals on a distal end of the cylindrical cutting head along a circumferential direction,
each of the plurality of blades is provided with a cutting edge selected from at least one of the following:
a cutting edge inclined along one direction, a cutting edge inclined along two directions, an arcuate cutting edge, or a cylindrical cutting edge.

4. The vascular anastomosis assembly according to claim 3, wherein the cutting head is disposed inside the main body portion of the vascular interface assembly, and an outer diameter of the cutting head is substantially equal to an inner diameter of the main body portion.

5. The vascular anastomosis assembly according to any one of claims 1 to 4, wherein the piercing assembly comprises a rod portion accommodated within a hollow rod of the cutting assembly, the piercing member is fixed at a distal end of the rod portion, and the piercing member comprises a blade forming a sharp distal end and a barb extending proximally; wherein
the blade of the piercing assembly is a three-piece blade or a four-piece blade; each piece in the three-piece blade comprises a cutting edge formed at a distal edge and a recessed, substantially flat portion formed at a proximal end; the four-piece blade comprises a main blade portion and positioning blade portions symmetrically arranged at both sides of the main blade portion, each of the positioning blade portions includes a cutting edge at a distal edge and a recessed, substantially flat portion disposed at a proximal end; or
the piercing member comprises an inverted conical main body portion, and the main body portion is provided with a plurality of slits along a circumferential direction, wherein each of the plurality of slits is embedded with a blade.

6. The vascular anastomosis assembly according to any one of claims 1 to 5, further comprising a release mechanism which is actuatable to release the vascular interface assembly from the applicator device, wherein the release mechanism preferably comprises a protrusion formed on a circumference of a distal end of the rod portion and a preferably L-shaped slot formed within a circumferential portion of the main body portion of the vascular interface assembly, and the protrusion is engaged into the slot.

7. The vascular anastomosis assembly according to any one of claims 1 to 6, wherein the clamping member is integrally formed with the main body portion, and the clamping member is placed in the expanded position by virtue of its own elasticity.

8. The vascular anastomosis assembly according to claim 7, wherein the clamping member comprises a plurality of claws arranged at intervals along a circumference of the main body portion, and each of the plurality of claws has an elongated shape, or has a rounded tip, or has a petal shape.

9. The vascular anastomosis assembly according to any one of claims 1 to 8, wherein the triggering member is disposed around the main body portion and is movable longitudinally, from a first position to a second position, along the main body portion; wherein
in the first position, the triggering member surrounds the clamping member to prevent the clamping member from being in the expanded position; and in the second position, the triggering member releases a restraint on the clamping member.

10. The vascular anastomosis assembly according to claim 9, wherein one of the triggering member and the main body portion is provided with a groove, and the other one of the triggering member and the main body portion is provided with a bump, and the groove is engaged with the bump to enable the triggering member to move along the main body portion.

11. The vascular anastomosis assembly according to claim 9, wherein the triggering member is provided with an internal thread, the main body portion is provided with an external thread, and the internal thread of the triggering member meshes with the external thread of the main body portion to enable the triggering member to rotate and move around the main body portion.

12. The vascular anastomosis assembly according to claim 10 or 11, further comprising a press ring,
wherein the press ring is operatively disposed at a distal end of the triggering member so as to move, without rotating, with the triggering member along the main body portion.

13. The vascular anastomosis assembly according to any one of claims 1 to 12, wherein a distal end of the main body portion is formed to be inclined, a distal end surface of the press ring or the triggering member is formed to be inclined, and an inclination angle ranges from 20° to 70°, preferably from 60° to 70°.

14. A vascular interface assembly, configured to be sealingly disposed in an incision of a blood vessel, comprising:
a cylindrical main body portion, an outer diameter of the main body portion being substantially equal to a diameter of the incision;
a synthetic vascular graft extending from a proximal end of the main body portion;
a clamping member disposed at a distal end of the main body portion, the clamping members being switchable between a retracted position and an expanded position;
a flange or a press ring disposed at a proximal end of the main body portion, wherein when the clamping member is in the expanded position, the vascular wall is sandwiched between the flange or the press ring and the clamping member; and
a triggering member, the triggering member being triggerable to switch the clamping member to the expanded position preferably by virtue of an elasticity of the clamping member.

15. The vascular interface assembly according to claim 14, wherein the triggering member is movable, between a first position and a second position, along the main body portion,
wherein in the first position, the clamping member is held in the retracted position; and in the second position, the clamping member is allowed to switch to the expanded position by virtue of an elasticity of the clamping member.

16. The vascular interface assembly according to claim 14 or 15, wherein the triggering member is integrally formed with the flange or the press ring, or the flange or the press ring moves with the triggering member.

17. The vascular interface assembly according to any one of claims 14 to 16, wherein a limiting member is disposed on an outer periphery of the main body portion, to limit a movement range of the triggering member or the press ring.

18. The vascular interface assembly according to any one of claims 14 to 17, wherein a distal end surface of the main body portion is formed to be inclined with an inclination angle which ranges from 20° to 70°, and preferably ranges from 60° to 70°.

19. The vascular interface assembly according to claim 18, wherein a distal end surface of the press ring is formed to be inclined with an inclination angle which ranges from 20° to 70°, and preferably ranges from 60° to 70°.
